# EUROPÄISCHE PATENTSCHRIFT

(11) **EP 3 863 500 B1**
(45) Veröffentlichungstag und Bekanntmachung des Hinweises auf die Patenterteilung: **17.01.2024**
(21) Anmeldenummer: 19801211.4
(22) Anmeldetag: 21.10.2019
(51) Int. Cl.: A61B 1/00, A61B 1/05, G02B 23/24, F16C 33/26

(54) **VIDEOENDOSKOP UND BREMSELEMENT**
VIDEO ENDOSCOPE AND BRAKE ELEMENT
VIDÉO-ENDOSCOPE ET ÉLÉMENT FREIN

(30) Priorität: 13.11.2018 DE 102018128306
(43) Veröffentlichungstag der Anmeldung: 18.08.2021
(73) Patentinhaber: OLYMPUS WINTER & IBE GMBH, 22045 Hamburg (DE)
(72) Erfinder: THÜMEN, Alrun, 22043 Hamburg (DE)
(74) Vertreter: Noack, Andreas
(86) Internationale Anmeldenummer: PCT/EP2019/078500
(87) Internationale Veröffentlichungsnummer: WO 2020/099067

(56) Entgegenhaltungen:
- EP-A1- 3 376 061
- DE-A1-102013 213 232
- US-A- 5 797 836
- US-A- 5 905 923
- US-A1- 2007 112 254

## Beschreibung

Die Erfindung betrifft ein Videoendoskop mit seitlicher Blickrichtung, umfassend einen langgestreckten Schaft mit einem proximalen Ende und einem distalen Ende, ein im Bereich des distalen Endes des Schafts angeordnetes Objektiv, einen proximal zu dem Objektiv angeordneten elektronischen Bildwandler, und einen am proximalen Ende des Schafts angeordneten Hauptkörper, wobei an dem Hauptkörper ein erstes Griffelement angeordnet ist, welches drehfest bezüglich des Schafts und des Hauptkörpers ausgeführt ist und dazu dient, den Schaft und den Hauptkörper um eine Längsachse des Schafts zu rotieren, um die Blickrichtung des Videoendoskops zu verändern; an dem Hauptkörper ein zweites Griffelement angeordnet ist, welches gegenüber dem Schaft und dem Hauptkörper um eine Längsachse des Schafts drehbar ausgeführt ist und dazu dient, eine Horizontlage des elektronischen Bildwandlers konstant zu halten, wenn die Blickrichtung des Videoendoskops durch Drehen des ersten Griffelements geändert wird; und zwischen dem zweiten Griffelement und dem Hauptkörper wenigstens ein ringförmiges elastisches Bremselement angeordnet ist.

Videoendoskope werden seit längerem in der Medizin eingesetzt, um schwer zugängliche Bereiche eines menschlichen oder tierischen Patienten optisch zu untersuchen und ggf. zu behandeln. Dazu weisen Videoendoskope in der Regel einen langgestreckten Schaft auf, welcher durch eine natürliche oder chirurgisch erzeugte Körperöffnung in den interessierenden Bereich eingeführt wird. Am proximalen Ende des Schafts ist ein Hauptkörper angeordnet, an welchem das Videoendoskop festgehalten und gesteuert werden kann. Am distalen Ende des Schafts ist ein Objektiv angeordnet, welches ein Bild des interessierenden Bereichs auf einen proximal zu dem Objektiv angeordneten elektronischen Bildwandler abbildet. Der Bildwandler erzeugt ein elektronisches Videosignal, welches über in dem Schaft verlaufende laufende Signalleitungen oder drahtlos zu dem Hauptkörper und von dort weiter zu einer Kontrolleinheit geleitet wird. Von der Kontrolleinheit kann das Videosignal zu einem Monitor und/oder einem Videoaufzeichnungsgerät geleitet werden.

Um den mit einem Videoendoskop beobachtbaren Bereich zu vergrößern weisen Videoendoskope oftmals eine seitliche Blickrichtung auf, also eine Blickrichtung, die von der Längsachse des Schafts um einen bestimmten Winkel abweicht. Durch Rotation des Schafts kann diese Blickrichtung gedreht werden.

Um bei einer Rotation des Schafts eine Horizontlage des Videobildes beizubehalten, muss der der elektronische Bildwandler in seiner rotatorischen Lage konstant gehalten werden. Hierzu weisen bekannte Videoendoskope am Hauptkörper zwei Griffelemente auf. Ein erstes Griffelement ist drehfest mit dem Schaft und dem Hauptkörper verbunden, so dass durch Drehen des ersten Griffelements der Schaft und somit die Blickrichtung des Videoendoskops rotiert werden kann. Das erste Griffelement kann beispielsweise einen am distalen Ende des Hauptkörpers angeordneten Drehring umfassen. Ein zweites Griffelement ist gegenüber dem ersten Griffelement und dem Hauptkörper drehbar ausgeführt und so mit dem Bildwandler gekoppelt, dass die rotatorische Ausrichtung des Bildwandlers stets der rotatorischen Ausrichtung des zweiten Griffelements entspricht.

Um nun die Blickrichtung des Videoendoskops zu ändern, kann der Benutzer das erste Griffelement mit einer Hand drehen und dabei das zweite Griffelement mit der zweiten Hand festhalten. Dadurch bleibt die Horizontlage des Videobildes stabil.

Um eine angenehme und sichere Bedienung des Videoendoskops zu gewährleisten, wird ein zur Drehung der Blickrichtung aufzuwendendes Drehmoment mittels eines ringförmigen elastischen Bremselements eingestellt, welches zwischen dem zweiten Griffelement und dem Hauptkörper und/oder dem ersten Griffelement angeordnet ist.

Zwischen gegeneinander drehbaren Teilen wie dem zweiten Griffelement und dem Hauptkörper und/oder dem ersten Griffelement entstehen zwangläufig Spalte, in welche während der Benutzung eines Videoendoskops Feuchtigkeit und Schmutz eindringen kann. Diese Spalte sind bei einer Aufbereitung des Videoendoskops nur schwer zu reinigen. Die eingesetzten ringförmigen elastischen Bremselemente können hier gleichfalls eine gewisse Dichtwirkung aufweisen, um ein Eindringen von Feuchtigkeit oder Schmutz zu vermeiden.

Entsprechende Videoendoskope werden beispielsweise von der Anmelderin unter der Bezeichnung "ENDOEYE" vertrieben.

Als ringförmige elastische Bremselemente werden im Stand der Technik zumeist Dichtringe aus einem Elastomer wie Gummi oder Silikon verwendet. Solche Dichtringe weisen eine hinreichende Beständigkeit gegen mechanische, thermische und chemische Belastungen auf, welche hauptsächlich bei der Aufbereitung von Videoendoskopen auf die Dichtringe wirken.

Die US 2007/0112254 A1 beschreibt ein Videoendoskop mit einem Handgriff und einem am Handgriff angeordneten Drehring zur Ausrichtung eines Bildaufnehmers bei Drehung des Videoendoskops. Zwischen dem Drehring und dem Handgriff sind Dichtungen vorgesehen.

Auch wenn die Dichtfunktion bekannter Elastomerdichtungen über viele Aufbereitungszyklen hinweg intakt bleibt, kommt es jedoch zu Veränderungen der Eigenschaften der Dichtringe. Insbesondere ist beobachtet worden, dass sich mit der Zeit die Gleiteigenschaften der Dichtringe verändern, wodurch die Drehung des zweiten Griffelements gegenüber dem Hauptkörper schwergängiger wird. Dies ist bei der Benutzung des Videoendoskops störend.

Es besteht daher die Aufgabe der Erfindung darin, ein Videoendoskop und ein ringförmiges Bremselement bereitzustellen, welche hinsichtlich der beschriebenen Problematik verbessert sind.

Diese Aufgabe wird gemäß eines Aspekts der Erfindung gelöst durch ein Videoendoskop mit seitlicher Blickrichtung, umfassend einen langgestreckten Schaft mit einem proximalen Ende und einem distalen Ende, ein im Bereich des distalen Endes des Schafts angeordnetes Objektiv, einen proximal zu dem Objektiv angeordneten elektronischen Bildwandler, und einen am proximalen Ende des Schafts angeordneten Hauptkörper, wobei an dem Hauptkörper ein erstes Griffelement angeordnet ist, welches drehfest bezüglich des Schafts und des Hauptkörpers ausgeführt ist und dazu dient, den Schaft und den Hauptkörper um eine Längsachse des Schafts zu rotieren, um die Blickrichtung des Videoendoskops zu verändern; an dem Hauptkörper ein zweites Griffelement angeordnet ist, welches gegenüber dem Schaft und dem Hauptkörper um eine Längsachse des Schafts drehbar ausgeführt ist und dazu dient, eine Horizontlage des elektronischen Bildwandlers konstant zu halten, wenn die Blickrichtung des Videoendoskops durch Drehen des ersten Griffelements geändert wird; und zwischen dem zweiten Griffelement und dem Hauptkörper und/oder dem ersten Griffelement wenigstens ein ringförmiges elastisches Bremselement angeordnet ist, welches dadurch weitergebildet ist, dass das wenigstens eine ringförmige elastische Bremselement eine in Umfangsrichtung gewickelte Schraubenfeder umfasst, deren Wicklungen so ausgerichtet sind, dass ein Flächenvektor einer durch eine einzelne Wicklung aufgespannte Fläche im Wesentlichen in Umfangsrichtung des Bremselements zeigt.

Die Erfindung schließt die Erkenntnis ein, dass sowohl die Gleiteigenschaften als auch die Dichtungseigenschaften der Schraubenfeder deutlich weniger durch mechanische, thermische und/oder chemische Einflüsse beeinträchtigt werden als die entsprechenden Eigenschaften einer Elastomerdichtung. Gleichzeitig hat sich überraschenderweise herausgestellt, dass die in einer in Umfangsrichtung gewickelten Schraubenfeder zwangsläufig auftreten Spalte die Dichtwirkung der Schraubenfeder gegen das Eindringen von Schmutz oder Feuchtigkeit nicht nennenswert beeinträchtigen.

In einer vorteilhaften Weiterbildung eines Videoendoskops gemäß der Erfindung kann die Schraubenfeder derart zwischen einer ersten Anlagefläche und einer zweiten Anlagefläche angeordnet sein, dass die Wicklungen der Schraubenfeder bei einer Drehung des zweiten Griffelements gegenüber dem Hauptkörper auf der ersten Anlagefläche und/oder der zweiten Anlagefläche entlanggleiten. Dadurch kommt die hohe Langzeitstabilität der Schraubenfeder unmittelbar dem Gleitverhalten des Videoendoskops zugute.

Dabei kann die Schraubenfeder aus leitfähigem Material, insbesondere Federstahl, bestehen, während die erste und/oder zweite Anlagefläche aus nicht leitendem Material, insbesondere Kunststoff, bestehen kann. Eine entsprechende Materialpaarung bietet einerseits besonders angenehme Gleiteigenschaften, und erlaubt andererseits eine elektrische Isolierung der Anlageflächen voneinander, ähnlich wie eine klassische Elastomerdichtung.

In einer Weiterbildung der Erfindung kann dem wenigstens einen ringförmigen Bremselement eine Labyrinth- oder Spaltdichtung vor- oder nachgeordnet sein. Solche zusätzlichen Bremselemente können die Dichtwirkung weiter verbessern, ohne die Gleiteigenschaften des Bremselements zu beeinflussen.

In einer vorteilhaften Ausgestaltung eines Videoendoskops nach der Erfindung können zwischen dem zweiten Griffelement und dem Hauptkörper und/oder dem ersten Griffelement zwei elastische Bremselemente angeordnet sein, welche jeweils eine in Umfangsrichtung gewickelte Schraubenfeder umfassen.

Dabei kann ein erstes elastisches Bremselement im Bereich eines proximalen Endes des Hauptkörpers angeordnet sein, und ein zweites elastisches Bremselement kann im Bereich eines distalen Endes des Hauptkörpers angeordnet sein. Auf diese Weise wird das zweite Griffelement auf dem Hauptkörper besonders sicher geführt. Außerdem lässt sich so ein Hohlraum zwischen dem Hauptkörper und dem zweiten Griffelement, welcher sich über die Länge des Hauptkörpers erstreckt, beidseitig abdichten.

In einer möglichen Ausführung eines Videoendoskops nach der Erfindung kann die Schraubenfeder einen unrunden Wicklungsquerschnitt weisen. Dabei kann ein größter Durchmesser des Wicklungsquerschnitts der Schraubenfeder in Längsrichtung des Schafts ausgerichtet sein. Ein kleinster Durchmesser des Wicklungsquerschnitts der Schraubenfeder kann senkrecht zur Längsachse des Schafts ausgerichtet sein.

Eine entsprechende Ausführung der Schraubenfeder hat verschiedene Vorteile. Zum einen verhindert die unrunde Form, dass die Schraubenfeder bei der Montage in Längsrichtung des Schafts rollt, wobei die Feder ungleich tordiert werden kann, was wiederum zu einer ungleichmäßigen Brems- und/oder Dichtwirkung führen würde. Gleichzeitig wird durch die geringeren Krümmungsradien an der Innen- und Außenseite der Schraubenfeder die Flächenpressung an den Anlageflächen reduziert.

In einer bevorzugten Weiterbildung eines Videoendoskops nach der Erfindung ist das wenigstens eine Bremselement in einem radialen Spalt angeordnet, wobei die radiale Höhe des Spaltes mindestens 10%, bevorzugt mindestens 25%, kleiner ist als der kleinste Durchmesser des Wicklungsquerschnitts der Schraubenfeder. Vorzugsweise beträgt die radiale Höhe des Spalts jedoch wenigstens 50% des kleinsten Durchmessers des Wicklungsquerschnitts der Schraubenfeder.

Durch die geringere Höhe des Spalts wird die Schraubenfeder senkrecht zur Längsachse des Schafts komprimiert, wobei die einzelnen Windungen der Schraubenfeder verkippt werden. Der Kippwinkel beträgt etwa 25°, wenn die Höhe des Spaltes 10% kleiner ist als der kleinste Durchmesser des Wicklungsquerschnitts der Schraubenfeder, und etwa 40°, wenn die Höhe des Spaltes 25% kleiner ist als der kleinste Durchmesser des Wicklungsquerschnitts der Schraubenfeder.

In einer weiteren vorteilhaften Ausgestaltung eines Videoendoskop nach der Erfindung ist das wenigstens eine Bremselement in einem radialen Spalt angeordnet, wobei die radiale Höhe des Spaltes weniger als 20%, bevorzugt weniger als 15%, besonders bevorzugt weniger als 10%, eines mittleren Radius des Spaltes beträgt. Durch eine entsprechende Dimensionierung des Spaltes ist sichergestellt, dass die Spalte zwischen den einzelnen Wicklungen der Schraubenfeder nicht so breit werden, dass die Dichtwirkung beeinträchtigt ist. Vorzugsweise beträgt die radiale Höhe des Spalts wenigstens 1% des mittleren Radius des Spaltes.

Die Schraubenfeder kann aus einem Draht mit im Wesentlichen runden Querschnitt gewickelt sein.

Die Erfindung wird nachfolgend anhand einiger beispielhafter Darstellungen näher beschrieben. Dabei dienen die dargestellten Ausführungsbeispiele ausschließlich dem besseren Verständnis der Erfindung, ohne diese auf die Ausführungsbeispiele zu beschränken.

Es zeigen:
Fig. 1: ein Videoendoskop,
Fig. 2: ein Videoendoskop in einer Schnittdarstellung,
Fig. 3a: ein elastisches Bremselement,
Fig. 3b: eine Ausschnittvergrößerung der Figur 3a,
Fig. 4: ein elastisches Bremselement im Schnitt,
Fig. 5a: ein Ausschnitt eines Bremselements in unbelastetem Zustand,
Fig. 5b+c: ein Ausschnitt eines Bremselements in belastetem Zustand.
Fig. 6: ein weiteres Videoendoskop in einer Schnittdarstellung,

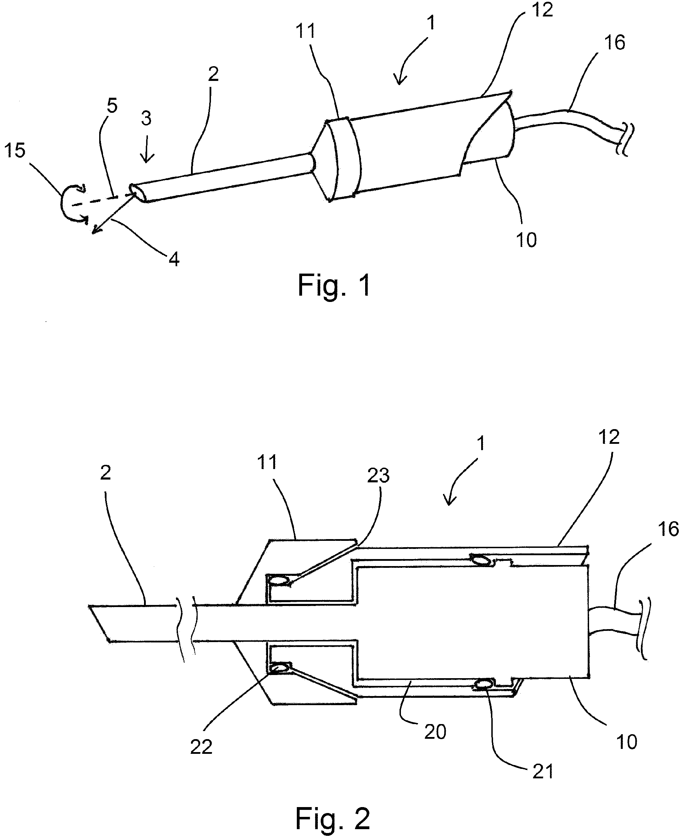

Figur 1 zeigt ein Videoendoskop 1 mit einem langgestreckten Schaft 2, in dessen distalem Ende ein Objektiv 3 angeordnet ist. Das Objektiv weist eine seitliche Blickrichtung auf, d.h., dass eine durch den Pfeil 4 angedeutete Blickrichtung des Objektivs 3 von einer Längsachse 5 des Videoendoskops 1 abweicht.

Weiterhin weist das Videoendoskop 1 einen Hauptkörper 10 auf, an dem ein erstes Griffelement 11 sowie ein zweites Griffelement 12 angeordnet sind.

Das erste Griffelement 11 ist als Drehrad am distalen Ende des Hauptkörpers ausgeführt. Mittels des ersten Griffelements 11 können der Schaft 2, das Objektiv 3, und der Hauptkörper 10 um die Längsachse 5 des Endoskops gedreht werden, so dass die Blickrichtung des Videoendoskops ebenfalls um die Längsachse 5 des Videoendoskops rotiert. Dies ist durch den Doppelpfeil 15 angedeutet.

In dem Schaft 2 ist proximal von dem Objektiv 3 ein nicht dargestellter elektronischer Bildwandler angeordnet, beispielsweise ein CCD-Chip oder ein CMOS-Chip. Natürlich können auch mehrere Bildwandler vorgesehen sein, um verschiedene Farbkanäle oder Teilbilder einer Stereooptik abzubilden. Der elektronische Bildwandler setzt das von dem Objektiv 3 abgebildete Bild in elektrische Signale um, welche durch den Schaft 2 in den Hauptkörper 10 und von dort über ein Kabel 16 an ein nicht dargestelltes externes Verarbeitungsgerät geleitet werden. Das Verarbeitungsgerät kann die elektrischen Signale beispielsweise zur Anzeige auf einem Monitor oder zum Abspeichern in einem Speicherelement aufbereiten.

Um bei einer Drehung der Blickrichtung des Videoendoskops 1 die Horizontlage des Videobildes konstant zu halten, ist der Bildwandler drehbar in dem Schaft 2 angeordnet, und mit dem zweiten Griffelement 12 drehgekoppelt. Dazu ist eine durch den Schaft oder durch die Wandung des Hauptkörpers 10 wirkende Magnetkupplung vorgesehen. Der Aufbau dieser Magnetkupplung ist an sich bekannt und braucht hier nicht näher erläutert werden.

Das zweite Griffelement 12 ist gegenüber dem ersten Drehkörper 11 und dem Hauptkörper 10 drehbar angeordnet. Wird nun die Blickrichtung des Videoendoskops durch Drehen des ersten Griffelements 11 rotiert, so kann gleichzeitig das zweite Griffelement 12 festgehalten werden, wodurch auch die Ausrichtung des elektronischen Bildwandler konstant bleibt.

In Figur 2 ist das Videoendoskop 1 in einer vereinfachten Schnittdarstellung gezeigt, wobei das Innere des Schafts 2 und des Hauptkörpers 10 der Übersichtlichkeit halber nicht dargestellt ist.

Es ist erkennbar, dass das erste Griffelement 11 im Bereich des Übergangs vom Schaft 2 in den Hauptkörper 10 drehfest mit dem Schaft 2 verbunden ist. Das zweite Griffelement 12 erstreckt sich vom proximalen Ende des Hauptkörpers 10 zu dessen distalem Ende, und verläuft dort zwischen dem Hauptkörper 10 und dem ersten Griffelement 11.

Um ein zur Drehung der Blickrichtung des Videoendoskops 1 erforderliches Drehmoment einzustellen, sind zwischen dem Hauptkörper 10 und dem zweiten Griffelement 12 sowie zwischen dem ersten Griffelement 11 und dem zweiten Griffelement 12 ringförmige, elastische Bremselemente 21, 22 vorgesehen, die weiter unten im Detail erläutert werden.

Zwischen dem Hauptkörper 10, dem ersten Griffelement 11, und dem zweiten Griffelement 12 entsteht ein Hohlraum 20, in welchen während der Benutzung des Videoendoskops 1 Feuchtigkeit und Schmutz eindringen können, die sich nur schwer entfernen lassen.

Die Bremselemente 21,22 wirken zusätzlich als Dichtungen, um das Eindringen von Feuchtigkeit und Schmutz in den Hohlraum 20 zu verhindern. Zusätzlich ist ein Spalt 23 zwischen dem ersten Griffelement 11 und dem zweiten Griffelement 12 besonders eng ausgeführt, so dass er als zusätzliche Spaltdichtung fungiert.

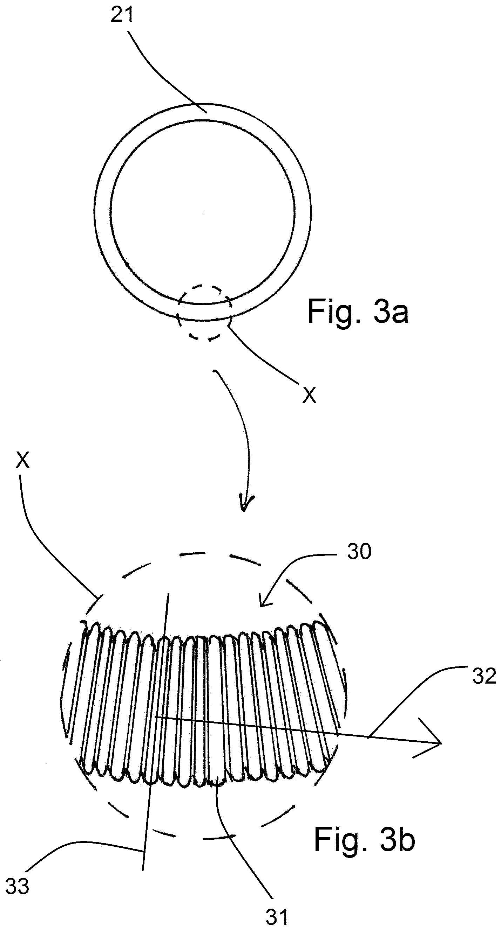

In Fig. 3a ist das elastische Bremselement 21 in unbelastetem Zustand in einer Vorderansicht dargestellt, Fig. 3b zeigt eine vergrößerte Ansicht eines Ausschnittes X der Figur 3a.

Es ist erkennbar, dass das Bremselement 21 aus einer Schraubenfeder 30 besteht. Die einzelnen Wicklungen 31 der Schraubenfeder 30 sind dabei so ausgerichtet, dass ein Flächenvektor 32 einer durch eine einzelne Wicklung 31 aufgespannte Fläche 33 im Wesentlichen in Umfangsrichtung des Bremselements 21 zeigt.

Die Schraubenfeder 30 ist aus einem Metalldraht mit im Wesentlichen runden Querschnitt gewickelt. Als Werkstoff für die Schraubenfeder 30 ist beispielsweise Edelstahl geeignet.

Zur Herstellung des ringförmigen Bremselements 21 kann die Schraubenfeder zunächst in der Umfangslänge des Bremselements als gerade Schraubenfeder gewickelt und anschließend elastisch zu einem Ring gebogen werden. Die Enden der Schraubenfeder können dann miteinander verbunden werden, beispielsweise verschweißt, so dass eine endlose, ringförmige Schraubenfeder entsteht.

Im Außenbereich des Bremselements 21 entstehen durch die Ringform zwangsläufig geringe Spalte zwischen den Wicklungen 31 der Schraubenfeder 30. Damit diese Spalte nicht so groß werden, dass die Dichtwirkung des Bremselements 21 beeinträchtigt wird, ist die radiale Höhe eines Spalts, in welchen das Bremselement 21 eingesetzt wird, deutlich kleiner als der mittlere Radius des Spalts. Vorzugsweise ist die Höhe des Spalts kleiner als 20%, besonders bevorzugt kleiner als 15%, und ganz besonders bevorzugt kleiner als 10% des mittleren Radius des Spalts.

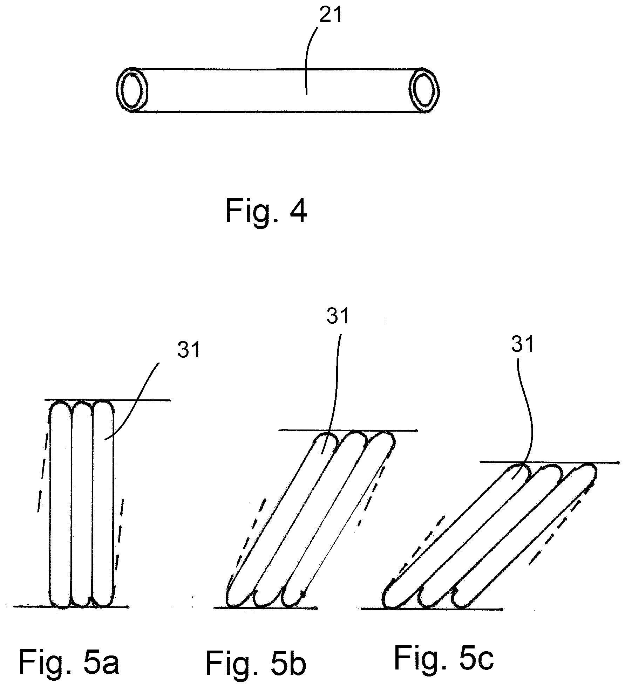

in Figur 4 ist das elastische Bremselement 21 in einem Querschnitt dargestellt. Es ist erkennbar, dass der Wicklungsquerschnitt des Bremselements 21 unrund, nämlich in etwa elliptisch ist. Dabei ist ein größter Durchmesser des Wicklungsquerschnitts in Richtung der Längsachse 5 des Schafts 2 ausgerichtet, während ein kleinster Durchmesser des Wicklungsquerschnitts senkrecht zur Längsachse 5 des Schafts 2 ausgerichtet ist.

Durch den unrunden Wicklungsquerschnitt des Bremselements 21 wird verhindert, dass das Bremselement 21 bei der Montage des Videoendoskops 1, bei der beispielsweise das zweite Griffelement 12 axial über den Hauptkörper 10 geschoben wird, entlang der Anlageflächen rollt. Hierbei könnte sich die Schraubenfeder 30 ungleichmäßig tordieren, so dass die Brems- und/oder Dichtwirkung des Bremselements 21 beeinträchtigt würde.

In den Figuren 5a bis 5c ist der Einfluss der Höhe eines Spaltes, in welchen die elastischen Bremselemente 21, 22 eingesetzt sind, auf die Struktur der Schraubenfeder 30 dargestellt.

Figur 5a zeigt die Lage der Wicklungen 31 der Schraubenfeder in entspanntem Zustand, hier verlaufen die Wicklungen 31 in etwa senkrecht zur Umfangsrichtung des Bremselements.

In Figur 5b ist die Situation dargestellt, wenn die Höhe des Spalts etwa 10% kleiner ist als der Wicklungsdurchmesser der Schraubenfeder 30. Es ist erkennbar, dass sich die Wicklungen 31 elastisch zur Seite legen, in der Figur 5b um etwa 25°. Dabei baut die Schraubenfeder 30 eine Rückstellkraft auf, welche für eine gleichmäßige und dichtende Anlage der Schraubenfeder 30 an die Anlageflächen sorgt.

Die Rückstellkraft der Schraubenfeder 30 bestimmt wesentlich die Reibung zwischen dem Bremselement 30 und den jeweiligen Anlageflächen und somit die vom Benutzer aufzuwendende Kraft beim Verdrehen des Blickwinkels des Videoendoskops 1. Da die Rückstellkraft kaum durch chemische oder thermische Belastungen beeinflusst wird, bleibt die Reibung über lange Zeit und über viele Aufbereitungszyklen konstant. Die genaue Rückstellkraft der Schraubenfeder 30, die für eine angenehme Handhabbarkeit des Videoendoskops 1 erforderlich ist, hängt stark von den Dimensionen des Videoendoskops 1 und den verwendeten Materialen ab. Sie kann für ein spezifisches Videoendoskop 1 ohne Schwierigkeiten durch einfache Experimente aufgefunden werden.

Bei einer Verdrehung des zweiten Griffelements 12 gegenüber dem Hauptkörper 10 des Videoendoskops 1 gleiten die Windungen 31 der Schraubenfeder 30 entweder auf der inneren Anlagefläche oder an der äußeren Anlagefläche entlang. Als eine besonders vorteilhafte Materialpaarung hat sich hier Stahl für die Schraubenfeder und Kunststoff, z.B. Polytetrafluorethylen (PTFE) oder Polyethylen (PE), für die Anlagefläche herausgestellt. Eine nichtleitende Anlagefläche bietet zusätzlich den Vorteil, dass die gegeneinander verdrehbaren Abschnitte des Videoendoskops (1) elektrisch gegeneinander isoliert sind, was den Verhältnissen bei der Verwendung klassischer Elastomerdichtungen entspricht.

In Figur 5c ist eine Situation dargestellt, bei der die Höhe des Spalts etwa 25% kleiner ist als der Wicklungsdurchmesser der Schraubenfeder 30. Hier liegen die Wicklungen 31 um einen Winkel von etwa 40° geneigt.

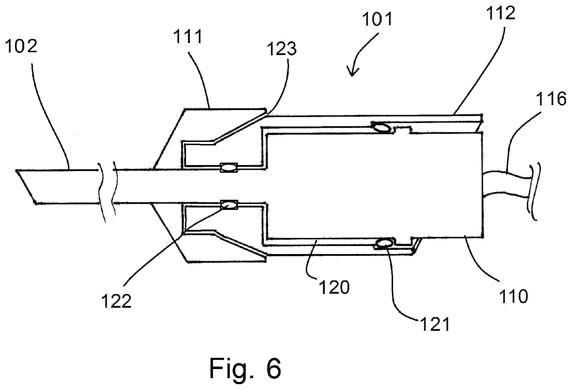

Die Ausführung des Bremselements 22 entspricht der beschriebenen Ausführung des Bremselements 21.ln Figur 6 ist eine alternative Ausführung eines Videoendoskops 101 dargestellt. Diese entspricht im Wesentlichen der in Figur 2 gezeigten Ausführung des Videoendoskops 1, wobei sich entsprechende Elemente mit einem um 100 erhöhten Bezugszeichen versehen und nicht erneut im Detail erläutert sind.

Anders als im Videoendoskop 1 ist im Videoendoskop 101 das zweite Bremselement 122 nicht zwischen dem ersten Griffelement 111 und dem zweiten Griffelement 112 angeordnet ist, sondern zwischen dem zweiten Griffelement 112 und dem Hauptkörper 110 im Bereich des Übergangs in den Schaft 102.

Als weitere Besonderheit sitzt das Bremselement 122 zwischen einer inneren Nut, die in dem Hauptkörper 110 eingebracht ist, und einer äußeren Nut, welche in das zweite Griffelement 112 eingebracht ist. Hierdurch lässt sich erreichen, dass bei der Montage des Videoendoskops 101 das Bremselement 122 quasi zwischen den beiden Nuten einrastet und somit die richtige Positionierung merkbar anzeigt. Eine entsprechende Ausführung kann selbstverständlich auch für das erste Bremselement 121 verwendet werden.

## Patentansprüche

1. Videoendoskop (1) mit seitlicher Blickrichtung, umfassend:
- einen langgestreckten Schaft (2) mit einem proximalen Ende und einem distalen Ende,
- ein im Bereich des distalen Endes des Schafts angeordnetes Objektiv (3),
- einen proximal zu dem Objektiv (3) angeordneten elektronischen Bildwandler, und
- einen am proximalen Ende des Schafts (2) angeordneten Hauptkörper (10),
wobei
- an dem Hauptkörper (10) ein erstes Griffelement (11) angeordnet ist, welches drehfest bezüglich des Schafts (2) und des Hauptkörpers (10) ausgeführt ist und dazu dient, den Schaft (2) und den Hauptkörper (10) um eine Längsachse (5) des Schafts (2) zu rotieren, um die Blickrichtung des Videoendoskops (1) zu verändern,
- an dem Hauptkörper (10) ein zweites Griffelement (12) angeordnet ist, welches gegenüber dem Schaft (2) und dem Hauptkörper (10) um eine Längsachse (5) des Schafts (2) drehbar ausgeführt ist und dazu dient, eine Horizontlage des elektronischen Bildwandlers konstant zu halten, wenn die Blickrichtung des Videoendoskops (1) durch Drehen des ersten Griffelements (11) geändert wird, und
- zwischen dem zweiten Griffelement (12) und dem Hauptkörper (10) und/oder dem ersten Griffelement (11) wenigstens ein ringförmiges elastisches Bremselement (21,22) angeordnet ist,
**dadurch gekennzeichnet, dass** das wenigstens eine ringförmige elastische Bremselement (21,22) eine in Umfangsrichtung gewickelte Schraubenfeder (30) umfasst, deren Wicklungen (31) so ausgerichtet sind, dass ein Flächenvektor (32) einer durch eine einzelne Wicklung (31) aufgespannte Fläche (33) im Wesentlichen in Umfangsrichtung des Bremselements (21) zeigt.

2. Videoendoskop (1) nach Anspruch 1, **dadurch gekennzeichnet, dass** die Schraubenfeder (30) derart zwischen einer ersten Anlagefläche und einer zweiten Anlagefläche angeordnet ist, dass die Wicklungen (31) der Schraubenfeder (30) bei einer Drehung des zweiten Griffelements (12) gegenüber dem Hauptkörper (10) auf der ersten Anlagefläche und/oder der zweiten Anlagefläche entlanggleiten.

3. Videoendoskop (1) nach Anspruch 1 oder 2, **dadurch gekennzeichnet, dass** die Schraubenfeder (30) aus leitfähigem Material, insbesondere Federstahl, besteht, und dass die erste und/oder zweite Anlagefläche aus nicht leitendem Material, insbesondere Kunststoff, besteht.

4. Videoendoskop (1) nach einem der Ansprüche 1 bis 3, **dadurch gekennzeichnet, dass** dem wenigstens einen ringförmigen Bremselement (21,22) eine Labyrinth- oder Spaltdichtung (23) vor- oder nachgeordnet ist.

5. Videoendoskop (1) nach einem der Ansprüche1 bis 4, **dadurch gekennzeichnet, dass** zwischen dem zweiten Griffelement (12) und dem Hauptkörper (10) und/oder dem ersten Griffelement (11) zwei ringförmige elastische Bremselemente (21,22) angeordnet sind, welche jeweils eine in Umfangsrichtung gewickelte Schraubenfeder (30) umfassen.

6. Videoendoskop (1) nach Anspruch 5, wobei ein erstes ringförmiges elastisches Bremselement (21) im Bereich eines proximalen Endes des Hauptkörpers (10) angeordnet ist, und wobei ein zweites ringförmiges elastisches Bremselement (22) im Bereich eines distalen Endes des Hauptkörpers (10) angeordnet ist.

7. Videoendoskop (1) nach einem der vorangehenden Ansprüche, **dadurch gekennzeichnet, dass** die Schraubenfeder (30) einen unrunden Wicklungsquerschnitt aufweist.

8. Videoendoskop (1) nach Anspruch 7, **dadurch gekennzeichnet, dass** ein größter Durchmesser des Wicklungsquerschnitts der Schraubenfeder (30) in Längsrichtung des Schafts (2) ausgerichtet ist.

9. Videoendoskop (1) nach Anspruch 6 oder 7, **dadurch gekennzeichnet, dass** ein kleinster Durchmesser des Wicklungsquerschnitts der Schraubenfeder (30) senkrecht zur Längsachse (5) des Schafts (2) ausgerichtet ist.

10. Videoendoskop (1) nach einem der vorangehenden Ansprüche, **dadurch gekennzeichnet, dass** das wenigstens eine Bremselement (21,22) in einem radialen Spalt angeordnet ist, wobei die radiale Höhe des Spaltes mindestens 10%, bevorzugt mindestens 25%, kleiner ist als der kleinste Durchmesser des Wicklungsquerschnitts der Schraubenfeder (30).

11. Videoendoskop (1) nach einem der vorangehenden Ansprüche, **dadurch gekennzeichnet, dass** das wenigstens eine Bremselement (21,22) in einem radialen Spalt angeordnet ist, wobei die radiale Höhe des Spaltes weniger als 20%, bevorzugt weniger als 15%, besonders bevorzugt weniger als 10%, eines mittleren Radius des Spaltes beträgt.

12. Videoendoskop (1) nach einem der vorangehenden Ansprüche, **dadurch gekennzeichnet, dass** die Schraubenfeder (30) aus einem Draht mit im Wesentlichen runden Querschnitt gewickelt ist.

## Claims

1. A video endoscope (1) with a lateral viewing direction, comprising:
- an elongated shaft (2) having a proximal end and a distal end,
- an objective lens (3) disposed in the portion of the distal end of the shaft,
- an electronic image converter disposed proximate to the objective lens (3), and
- a main body (10) disposed at the proximal end of the shaft (2),
wherein
- a first grip element (11) is disposed on the main body (10), the first grip element (11) being rotationally fixed with respect to the shaft (2) and the main body (10) and serving to rotate the shaft (2) and the main body (10) about a longitudinal axis (5) of the shaft (2) in order to change the viewing direction of the video endoscope (1),
- a second grip element (12) is disposed on the main body (10), the second grip element (12) being rotatable relative to the shaft (2) and the main body (10) about a longitudinal axis (5) of the shaft (2) and serving to maintain a horizontal position of the electronic image converter when the viewing direction of the video endoscope (1) is changed by rotating the first grip element (11), and
- between the second grip element (12) and the main body (10) and/or the first grip element (11) at least one annular elastic brake element (21, 22) is disposed, **characterized in that** the at least one annular elastic brake element (21, 22) comprises a circumferentially wound coil spring (30), the windings (31) of which are oriented such that a surface vector (32) of a surface (33) spanned by a single winding (31) points substantially in the circumferential direction of the brake element (21).

2. Video endoscope (1) according to claim 1, **characterized in that** the coil spring (30) is disposed between a first contact surface and a second contact surface in such a way that the windings (31) of the coil spring (30) slide along the first contact surface and/or the second contact surface during a rotation of the second grip element (12) relative to the main body (10).

3. Video endoscope (1) according to claim 1 or 2, **characterized in that** the coil spring (30) is made of conductive material, in particular spring steel, and **in that** the first and/or second contact surface is made of non-conductive material, in particular plastic.

4. Video endoscope (1) according to one of claims 1 to 3, **characterized in that** a labyrinth seal or gap seal (23) is disposed in front of or behind the at least one annular brake element (21, 22).

5. Video endoscope (1) according to one of claims 1 to 4, **characterized in that** between the second grip element (12) and the main body (10) and/or the first grip element (11) two annular elastic brake elements (21, 22) are disposed, each of which comprises a coil spring (30) wound in the circumferential direction.

6. Video endoscope (1) according to claim 5, wherein a first annular elastic brake element (21) is disposed in a portion of a proximal end of the main body (10), and wherein a second annular elastic brake element (22) is disposed in a portion of a distal end of the main body (10).

7. Video endoscope (1) according to one of the preceding claims, **characterized in that** the coil spring (30) has a non-circular coil cross-section.

8. Video endoscope (1) according to claim 7, **characterized in that** a largest diameter of the coil cross-section of the coil spring (30) is aligned in the longitudinal direction of the shaft (2).

9. Video endoscope (1) according to claim 6 or 7, **characterized in that** a smallest diameter of the coil cross-section of the coil spring (30) is aligned perpendicular to the longitudinal axis (5) of the shaft (2).

10. Video endoscope (1) according to one of the preceding claims, **characterized in that** the at least one brake element (21, 22) is disposed in a radial gap, the radial height of the gap being at least 10%, preferably at least 25%, smaller than the smallest diameter of the coil cross-section of the coil spring (30).

11. Video endoscope (1) according to one of the preceding claims, **characterized in that** the at least one brake element (21, 22) is arranged in a radial gap, the radial height of the gap being less than 20%, preferably less than 15%, particularly preferably less than 10%, of a mean radius of the gap.

12. Video endoscope (1) according to any one of the preceding claims, **characterized in that** the coil spring (30) is wound from a wire having a substantially circular cross-section.

## Revendications

1. Vidéo-endoscope (1) à vision latérale, comprenant :
- une tige allongée (2) avec une extrémité proximale et une extrémité distale,
- un objectif (3) disposé dans la zone de l'extrémité distale de la tige
- un convertisseur d'images électronique disposé à proximité de l'objectif (3), et
- un boîtier principal (10) disposé à l'extrémité proximale de la tige (2),
où
- sur le boîtier principal (10) est disposé un premier élément de poignée (11) qui est réalisé de manière à ne pas pouvoir tourner par rapport à la tige (2) et au boîtier principal (10) et qui sert à faire tourner la tige (2) et le boîtier principal (10) autour d'un axe longitudinal (5) de la tige (2) afin de modifier la direction de vision du vidéo-endoscope (1),
- sur le boîtier principal (10) est disposé un deuxième élément de poignée (12) qui est réalisé de manière à pouvoir tourner par rapport à la tige (2) et au boîtier principal (10) autour d'un axe longitudinal (5) de la tige (2) et qui sert à maintenir constante une position horizontale du convertisseur d'images électronique lorsque la direction de vision du vidéoendoscope (1) est modifiée par rotation du premier élément de poignée (11), et
- au moins un élément de freinage élastique annulaire (21, 22) est disposé entre le deuxième élément de poignée (12) et le boîtier principal (10) et/ou le premier élément de poignée (11),
**caractérisé en ce que** le au moins un élément de freinage élastique annulaire (21, 22) comprend un ressort hélicoïdal (30) enroulé dans la direction circonférentielle, dont les enroulements (31) sont orientés de telle sorte qu'un vecteur de surface (32) d'une surface (33) tendue par un seul enroulement (31) pointe essentiellement dans la direction circonférentielle de l'élément de freinage (21).

2. Vidéo-endoscope (1) selon la revendication 1, **caractérisé en ce que** le ressort hélicoïdal (30) est disposé entre une première surface d'appui et une deuxième surface d'appui de telle sorte que les enroulements (31) du ressort hélicoïdal (30) glissent le long de la première surface d'appui et/ou de la deuxième surface d'appui lors d'une rotation du deuxième élément de préhension (12) par rapport au boîtier principal (10).

3. Vidéo-endoscope (1) selon la revendication 1 ou 2, **caractérisé en ce que** le ressort hélicoïdal (30) est constitué d'un matériau conducteur, notamment d'acier à ressort, et **en ce que** la première et/ou la deuxième surface d'appui sont constituées d'un matériau non conducteur, notamment de matière plastique.

4. Vidéo-endoscope (1) selon l'une des revendications 1 à 3, **caractérisé en ce qu'**un joint à labyrinthe ou à fente (23) est placé en amont ou en aval d'au moins un élément de freinage annulaire (21, 22).

5. Vidéo-endoscope (1) selon l'une des revendications 1 à 4, **caractérisé en ce que** deux éléments de freinage élastiques annulaires (21, 22) sont disposés entre le deuxième élément de poignée (12) et le boîtier principal (10) et/ou le premier élément de poignée (11), lesquels comprennent chacun un ressort hélicoïdal (30) enroulé dans la direction circonférentielle.

6. Vidéo-endoscope (1) selon la revendication 5, dans lequel un premier élément de freinage élastique annulaire (21) est disposé dans la zone d'une extrémité proximale du boîtier principal (10), et dans lequel un deuxième élément de freinage élastique annulaire (22) est disposé dans la zone d'une extrémité distale du boîtier principal (10).

7. Vidéo-endoscope (1) selon l'une des revendications précédentes, **caractérisé en ce que** le ressort hélicoïdal (30) présente une section d'enroulement non circulaire.

8. Vidéo-endoscope (1) selon la revendication 7, **caractérisé en ce qu'**un diamètre maximal de la section transversale d'enroulement du ressort hélicoïdal (30) est orienté dans la direction longitudinale de la tige (2).

9. Vidéo-endoscope (1) selon la revendication 6 ou 7, **caractérisé en ce qu'**un plus petit diamètre de la section transversale d'enroulement du ressort hélicoïdal (30) est orienté perpendiculairement à l'axe longitudinal (5) de la tige (2).

10. Vidéo-endoscope (1) selon l'une des revendications précédentes, **caractérisé en ce que** l'au moins un élément de freinage (21, 22) est disposé dans un interstice radial, la hauteur radiale de l'interstice étant inférieure d'au moins 10%, de préférence d'au moins 25%, au plus petit diamètre de la section transversale d'enroulement du ressort hélicoïdal (30).

11. Vidéo-endoscope (1) selon l'une des revendications précédentes, **caractérisé en ce que** l'au moins un élément de freinage (21, 22) est disposé dans un interstice radial, la hauteur radiale de l'interstice étant inférieure à 20%, de préférence inférieure à 15%, de manière particulièrement préférée inférieure à 10%, d'un rayon moyen de l'interstice.

12. Vidéo-endoscope (1) selon l'une des revendications précédentes, **caractérisé en ce que** le ressort hélicoïdal (30) est enroulé à partir d'un fil ayant une section transversale sensiblement ronde.
